(19) **Europäisches Patentamt**
**European Patent Office**
**Office européen des brevets**

(11) **EP 2 356 579 B1**

(12) **EUROPEAN PATENT SPECIFICATION**

(45) Date of publication and mention
of the grant of the patent:
**20.05.2015 Bulletin 2015/21**

(51) Int Cl.:
**G06N 7/00** *(2006.01)*     *G06F 15/18 (2006.01)*

(21) Application number: **09825222.4**

(86) International application number:
**PCT/US2009/062119**

(22) Date of filing: **26.10.2009**

(87) International publication number:
**WO 2010/053743 (14.05.2010 Gazette 2010/19)**

(54) **LONG TERM ACTIVE LEARNING FROM LARGE CONTINUALLY CHANGING DATA SETS**

LANGZEIT-AKTIVLERNEN AUS GROSSEN SICH KONTINUIERLICH ÄNDERNDEN
DATENSÄTZEN

APPRENTISSAGE ACTIF À LONG TERME À PARTIR DE GRANDS ENSEMBLES DE DONNÉES
CHANGEANT CONTINUELLEMENT

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **29.10.2008   US 109490 P
03.04.2009   US 166472 P
03.04.2009   US 166486 P
03.04.2009   US 166499 P
19.10.2009   US 252978 P**

(43) Date of publication of application:
**17.08.2011   Bulletin 2011/33**

(73) Proprietor: **The Regents of the University of
Colorado, a body
corporate
Denver, CO 80203 (US)**

(72) Inventors:
• **GRUDIC, Gregory Zlatko
Longmont
Colorado 80503 (US)**
• **MOULTON, Steven Lee
Littleton
Colorado 80121 (US)**
• **MULLIGAN, Isobel Jane
Niwot, CO 80503 (US)**

(74) Representative: **Jansen, Cornelis Marinus et al
V.O.
Johan de Wittlaan 7
2517 JR Den Haag (NL)**

(56) References cited:
**WO-A1-2007/098957     US-A1- 2003 125 612
US-A1- 2003 176 931     US-A1- 2003 200 189
US-A1- 2003 212 678     US-A1- 2006 161 403
US-A1- 2008 133 434     US-A1- 2008 154 814**

• **W. C. SHOEMAKER ET AL: "Outcome Prediction
of Emergency Patients by Noninvasive
Hemodynamic Monitoring", CHEST, vol. 120, no.
2, 1 August 2001 (2001-08-01), pages 528-537,
XP55029137, ISSN: 0012-3692, DOI:
10.1378/chest.120.2.528**
• **MICHAEL J PROCOPIO ET AL: "Learning in
dynamic environments with Ensemble Selection
for autonomous outdoor robot navigation",
INTELLIGENT ROBOTS AND SYSTEMS, 2008.
IROS 2008. IEEE/RSJ INTERNATIONAL
CONFERENCE ON, IEEE, PISCATAWAY, NJ,
USA, 22 September 2008 (2008-09-22), pages
620-627, XP031348747, ISBN: 978-1-4244-2057-5**

**Description**

BACKGROUND OF THE INVENTION

This application relates generally to methods and systems of active learning. More specifically, this application relates to long-term active learning from large continually changing data sets, including the autonomous development of predictive models. This application also relates to methods and systems that apply active learning models to predict specific out comes. These outcomes can be in the medical, military, and/or robotics arenas, to name a few.

There are numerous applications in which active-learning techniques are needed, ranging among medical applications, engineering applications, manufacturing applications and others. Examples of such active-learning techniques include expert-system techniques, iterative techniques, neural-network techniques and genetic algorithms, among others.

An expert system essentially uses a machine to reproduce the performance of human experts. It typically relies on the creation of a knowledgebase, that uses a knowledge-representation formalism to capture the knowledge of subject-matter experts. The knowledgebase is populated by gathering the relevant knowledge from the subject-matter experts and codifying it according to the representation formalism. Commonly, a learning component is included so that the content of the knowledgebase may be modified as the expert system is used in the same real-world problem-solving circumstances as are considered by the subject-matter experts, thereby improving its performance.

Iterative techniques begin with a seed solution to a defined problem that is processed by a formalism to produce a result that is compared with an observed result. If the formal result differs by more than a defined amount from the observed result, the solution is modified and reprocessed by the formalism. Various techniques are applied so that the modifications of the solution are driven towards converging the formal result with the observed result. When the convergence is achieved at a satisfactory level, the solution is taken as well approximating the real-world conditions that produced the observed result.

Neural networks typically include a plurality of nodes, with each node having a weight value associated with it. One layer of nodes is an input layer that has a plurality of input nodes and another layer of nodes is an output layer that has a plurality of output nodes, with at least one intermediate layer of nodes there between. Input data are provided to the layer of input nodes and the weight values applied by the network to generate results at the layer of output nodes. To train the neural network, the resulting output values are compared against correct interpretations of known samples. If the output value in such a comparison is incorrect, the network modifies itself to arrive at the correct value. This is achieved by connecting or disconnecting certain nodes and/or adjusting the weight values of the nodes during the training. Once the training is completed, the resulting layer/node configuration and corresponding weights represent a trained neural network, which is then ready to receive unknown data and make interpretations based on the data. Self learning and/or predictive models that can handle large amounts of possibly complex, continually changing data have not been described or successfully implemented for medical care.

Appropriate resuscitation of an injured patient demands an accurate assessment of physical exam findings, correct interpretation of physiological changes and an understanding of treatment priorities. Resuscitative trauma care is provided by a broad range of individuals with varying levels of interest and experience. It can require a large amount of information be quickly gathered, accurately interpreted and meaningfully conveyed to a coordinated group of local and downstream healthcare providers.

Traumatic brain injury (TBI) and exsanguination are the two most common causes of death during the resuscitative phase of trauma care. The management of head injury, hemorrhage and fluid resuscitation are therefore integral parts of early trauma care.

Traumatic brain injury (TBI) is a common and devastating condition. It is the number one cause of death and disability in the pediatric population, affecting over half a million children annually in the U.S. TBI accounts for approximately 60,000 adult and pediatric deaths in the U.S. each year. TBI outcome depends on the severity of primary brain injury (direct injury to the brain due to mechanical insult) and the effectiveness of preventing or limiting secondary brain injury (defined as damage to the brain due to the body's physiological response to the initial mechanical insult). The cranium is a bony compartment with a fixed volume. Following head trauma, blood vessels within and around the brain may rupture and bleed into the brain (causing intracerebral hemorrhage) and/or around the brain (causing development of an epidural and/or subdural hematoma to form). Bleeding in this fashion compresses the brain. The brain also swells as a result of injury. These types of secondary injury increase the intracranial pressure and decrease cerebral perfusion, leading to brain ischemia. Brain ischemia causes further brain swelling, more ischemia and if not treated and managed appropriately, brain herniation through the base of the skull (where the spinal cord exits) and death.

Evidence based guidelines for the management of severe traumatic brain injury have been developed, yet a wide spectrum of methods still characterizes most monitoring and treatment strategies. The most widely used, current method for intracranial pressure monitoring involves placement of an intracranial pressure monitoring device. This is an invasive procedure that involves cutting the scalp and drilling a hole through the patient's cranium, so that a pressure transducer can be inserted in or on top of the brain. Newer, non-invasive methods for intracranial pressure and cerebral perfusion monitoring have been described; however, these methods are still considered experimental and none are in clinical practice. These non-invasive, intracranial pressure monitoring methods include: transcranial Doppler ultrasonography; transcranial optical radiation, such as near-infrared spectroscopy; ophthalmodynamometry; arterial pulse phase lag; and ocular coherence tomography.

Posttraumatic seizure (PTS) is associated with severe primary brain injury and, importantly, could itself also act as a type of secondary brain injury. Electrographic only posttraumatic seizures, which can be seen in up to 45% of pediatric moderate-severe TBI patients, have been shown to cause elevated ICP and metabolic stress. Moreover, posttraumatic seizures (occurring $\leq$ 7 days post-injury) have been shown to negatively impact outcome and increase morbidity. Thus, posttraumatic seizure is a potential therapeutic target and one of the few potentially preventable causes of secondary brain injury following TBI.

It is difficult to identify at-risk patients who will benefit from early anti-seizure prophylaxis and prevention of acute secondary brain injury. Clinical markers, such as mental status and seizure-like movements, can be monitored; however, these markers of PTS are often masked by altered mental status/coma, sedatives and paralytics, and even anticonvulsants. Continuous electroencephalographic (cEEG) monitoring in moderate-severe TBI has been shown in the adult literature to increase PTS detection rates by 22-33%. This is a labor intensive method requiring the collection of visual and continuous 21 channel EEG data. This large volume of data must then be reviewed by a trained epileptologist. Further, it is unclear which of the available anticonvulsants are most useful in adults and children, based on antiepileptic effect, antiepileptogenic effects, duration of treatment, and effect on outcome.

Prior research has been done on the automated identification of seizures in cEEG data, achieving detection rates of 70-80% and 1-3 false positives per hour, but the work has not yet yielded a product or prototype. These systems have typically been rule-based, where a set of feature detectors are combined using thresholds and qualitative or quantitative constraints.

Fluid resuscitation strategies are poorly understood, difficult to study and variably practiced. Inadequate resuscitation poses the risk of hypotension and end organ damage. Conversely, aggressive fluid resuscitation may dislodge clots from vascular injuries, resulting in further blood loss, hemodilution and death. How to best proceed when one is dealing with a multiply-injured patient who has a traumatic brain injury *and* exsanguinating hemorrhage can be especially difficult. Under resuscitation can harm the already injured brain, whereas over resuscitation can reinitiate intracranial bleeding and exacerbate brain swelling, leading to brain herniation, permanent neurological injury and oftentimes death.

US2003125612 discloses medical monitoring physiological characteristics values. Dynamic glucose monitoring functions are provided that perform predictive analysis. Results of this analysis are compared with one or more thresholds. Default thresholds are used when no user setting is provided, or the thresholds can be set according to a time schedule.

Shoemaker et al have published an article titled "Outcome prediction of emergency patients by noninvasive hemodynamic monitoring" in Chest 2001 (EPO reference XP 55029137). This article discloses use of non-invasive measurements on patients to prospectively estimate circulatory patterns in patients.

Procopio et al have published an article titled "Learning in Dynamic Environments with Ensemble Selection for Outdoor Robot Navigation" in the 2008 IEEE conference on intelligent robots and systems (Nice, France September 22-26, 2008) pages 620-627. The article considers the problem of finding safe paths for a robot and proposes to use ensemble selection to combine models of time evolving data associated with the robot domain.

BRIEF SUMMARY OF THE INVENTION

Embodiments of the invention can be implemented to use high dimensional, complex domains, where large amounts of variable, possibly complex data exist on a continuous, and/or possibly dynamically changing timeline. Various embodiments can be implemented in disparate fields of endeavor. For example, embodiments of the invention can be implemented in the fields of robotics and medicine. In the field of robotics, embodiments of the invention can use real-time image (and information derived from other sensors modalities) analysis, high speed data processing and highly accurate decision-making to enable robot navigation in outdoor, unknown unstructured environments. Embodiments of the invention can also be applied to physiological (vital sign) and clinical data analysis in the field of medicine. In such embodiments, an algorithm can discover and model the natural, complex, physiological and clinical relationships that exist between normal, injured and/or diseased organ systems, to accurately predict the current and future states of a patient.

In some embodiments of the invention methods are provided for autonomously building a predictive model of outcomes. A most-predictive set of signals $S_k$ can be autonomously identified out of a set of signals $s_1, s_2, ..., s_D$ for each of one or more outcomes $o_k$. A set of probabilistic predictive models $\hat{o}_k = M_k(S_k)$ can be autonomously learned, where $\hat{o}_k$ is a prediction of outcome $o_k$ derived from the model $M_k$ that uses as inputs values obtained from the set of signals $S_k$. The step of autonomously learning can be repeated incrementally from data that contains examples of values of signals $s_1, s_2, ..., s_D$ (possibly dynamically changing) and corresponding outcomes $o_1, o_2, ..., o_K$.

In some embodiments autonomously learning can include using a linear model framework to identify predictive variables for each increment of data. The linear model framework may be constructed with the form

$$\hat{o}_k = f_k\left(a_0 + \sum_{i=1}^{d} a_i s_i\right),$$ where $f_k$ is any mapping from one input to one output and $a_0, a_1, ..., a_d$ are linear model coefficients. In some embodiments, autonomously learning can include determining or estimating which signals are not predictive from the set of signals and outcomes. The corresponding coefficients for these signals can then be set to 0. An autonomous learning method can then build a predictive density model using these predictive coefficients, signals, and/or outcomes. In some embodiments, the method can repeat each time a new signal outcome pair is received or encountered that is predictive.

Embodiments of the invention also provide methods for predicting volume of acute blood loss from a patient. Data values are collected from one or more physiological sensors attached to the patient. A hemodynamic compensation model is applied to the collected data values to predict the volume of acute blood loss from the patient. The hemodynamic compensation model can be previously generated from a plurality of data values collected from physiological sensors attached to a plurality of subjects.

Embodiments of the invention can also provide methods for predicting volume of acute blood loss from a patient that will cause hemodynamic decompensation, also termed cardiovascular (CV) collapse. Data values are collected from one or more physiological sensors attached to the patient. A hemodynamic compensation model is applied to the collected data values to predict the volume of acute blood loss from the patient that will cause CV collapse. The hemodynamic compensation model can be previously generated from a plurality of data values collected from physiological sensors attached to a plurality of subjects.

In some embodiments, the one or more physiological sensors may comprise an electrocardiograph, a pulse oximeter, a transcranial Doppler sensor, or a capnography sensor, among others. The collected data values may include a photoplethysmograph, a perfusion index, a pleth variability index, cardiac output, heart stroke volume, arterial blood pressure, systolic pressure, diastolic blood pressure, mean arterial pressure, systolic pressure variability, pulse pressure, pulse pressure variability, stroke volume, cardiac index, or near-infrared spectroscopy data, among others.

Embodiments of the invention also provide methods for determining brain pressures within a subject. A plurality of parameters are measured from the subject. The parameters are applied to a model that relates the parameters to various brain pressures, with the model having been derived from application of a machine-learning algorithm. This allows the brain pressures to be determined from the model.

The brain pressure may comprise an intracranial pressure or a cerebral perfusion pressure in different embodiments. The plurality of parameters may comprise heart rate, systolic blood pressure, diastolic blood pressure, mean arterial pressure, cardiac output, pulse oximetry data, carotid blood flow, among others.

Embodiments of the invention also provide methods detecting seizures based on continuous EEG waveform data from a subject. A plurality of parameters can be derived from cEEG data measured from the subject. The parameters are applied to a model that relates the parameters to seizure wavefrom activity, with the model having been derived from application of a machine-learning algorithm. This allows seizure activity to be determined from the model.

Autonomous learning methods, robot navigation methods, acute blood loss determination methods, prediction of CV collapse, brain pressure determination methods and detection, as well as predicition, of seizure activity can be embodied on a system having an input device and a processor provided in electrical communication with the input device. The processor can include a computer-readable storage medium that includes instructions for implementing the method as described.

BRIEF DESCRIPTION OF THE DRAWINGS

A further understanding of the nature and advantages of the present invention may be realized by reference to the remaining portions of the specification and the drawings.

FIG. 1 is a schematic block diagram illustrating the structure of a computer system on which methods of the invention may be embodied.

FIG. 2 is a flow diagram that summarizes various methods of the invention.

FIG. 3 is a schematic diagram illustrating a basic structure for embodiments of the invention.

FIG. 4 is a flow diagram summarizing methods of the invention in certain embodiments.

FIG. 5 is a flow diagram that summarizes various embodiments of the invention.

FIG. 6 is a graph showing algorithmic predicted level of predicted level of lower body negative pressure (LBNP) and the LBNP that will cause cardiovascular collapse during LBNP experiments.

FIG. 7 is a graph of predicted blood volume approaching the predicted point of CV collapse using embodiments of the present invention.

DETAILED DESCRIPTION OF THE INVENTION

Embodiments of the invention provide methods and systems for autonomously building predictive models of current and future outcomes using large amounts of possibly complex, continually changing, incrementally available data. A general predictive model is disclosed followed by specific augmentation to the predictive model in specific applications. Prior to describing the predictive model, an example of a computational device is disclosed that can be used to implement various embodiments of the invention. Following the description of the predictive model, specific embodiments are disclosed implementing the predictive model in various aspects.

Embodiments of the invention provide methods and systems for autonomously building predictive models of current and future outcomes, using large amounts of possibly complex, continually changing, incrementally available data. Such embodiments find application in a diverse range of applications. Merely by way of illustration, some exemplary applications include autonomous robot navigation in unknown, outdoor unstructured, environments; a human hemorrhaging model for the continuous, noninvasive detection of acute blood loss; and/or a human hemorrhaging model for fluid resuscitation and the prediction of cardiovascular collapse and intracranial pressure. Such examples are not intended to limit the scope of the invention, which is more generally suitable for any application in which current and future outcomes are desired to be known on the basis of large, continually changing datasets.

**Computation Device**

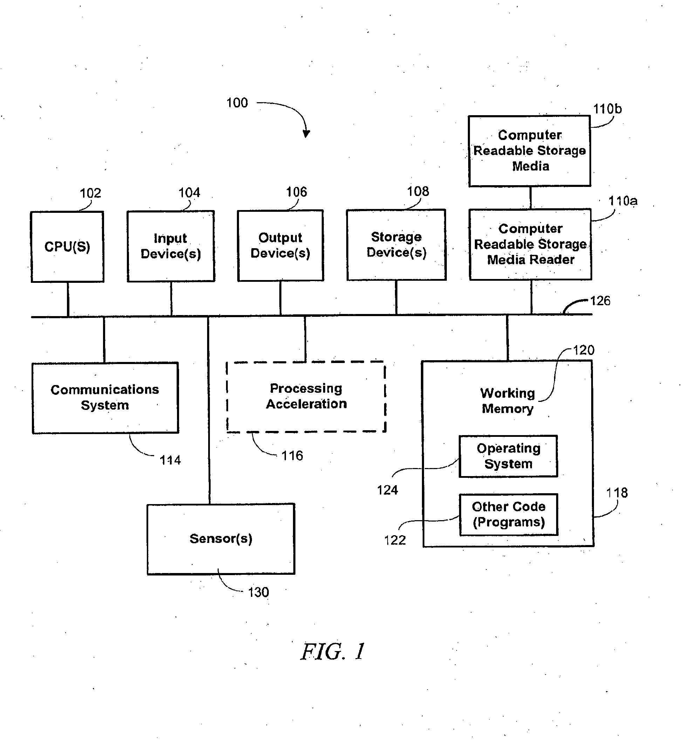

The predicative and/or self learning models may be embodied on computation devices, a typical structure for which is shown schematically in FIG. 1. This block diagram broadly illustrates how individual system elements may be implemented in a separated or more integrated manner. The computational device 100 is shown comprised of hardware elements that are electrically coupled via bus 126, including a host processor 102, an input device 104, an output device 106, a storage device 108, a computer-readable storage media reader 110a, a communications system 114, a processing acceleration unit 116 such as a DSP or special-purpose processor, and a memory 118. The computer-readable storage media reader 110a is further connected to a computer-readable storage medium 110b, the combination comprehensively representing remote, local, fixed, and/or removable storage devices plus storage media for temporarily and/or more permanently containing computer-readable information. The communications system 114 may comprise a wired, wireless, modem, and/or other type of interfacing connection and permits data to be exchanged. Sensor connection 130 can be included that can be used to couple with a sensor or other data input device. Sensor interface 130, in some embodiments, can input data for real time processing. In other embodiments, sensor interface 130 can input data into storage devices 108 for processing at a later time. Any type of sensor can be used that provides input data signals and/or outcomes. Various sensors are described throughout this disclosure and can be coupled with computational device 100.

Computational device 100 can also include software elements, shown as being currently located within working memory 120, including an operating system 124 and other code 122, such as a program designed to implement methods of the invention such as predictive and/or self learning algorithms disclosed throughout the specification. It will be apparent to those skilled in the art that substantial variations may be made in accordance with specific requirements. For example, customized hardware might also be used and/or particular elements might be implemented in hardware, software (including portable software, such as applets), or both. Further, connection to other computing devices such as network input/output devices may be employed.

**A Self-Learning Predictive Model**

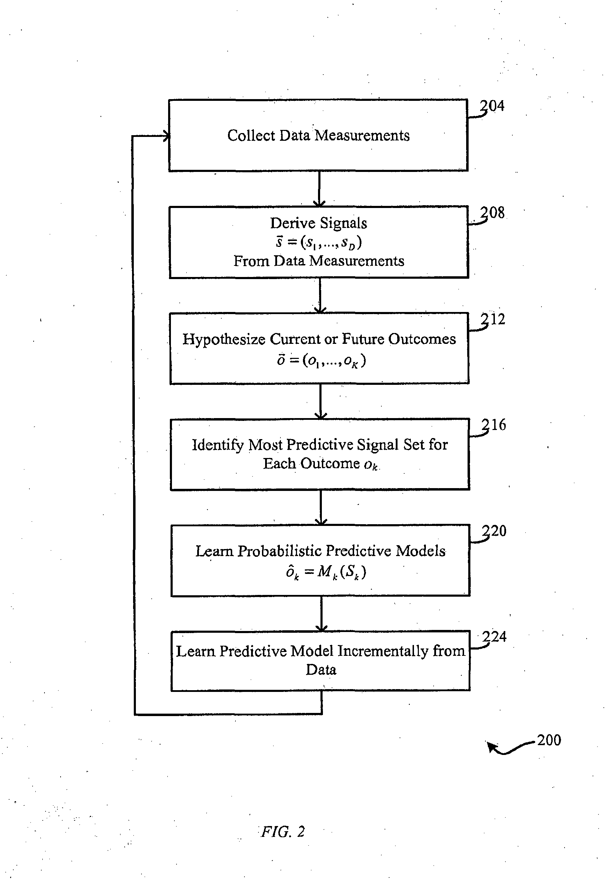

A self-learning predictive model (or machine learning) method is provided with the flow diagram 200 of FIG. 2 according to some embodiments of the invention. Method 200 begins at block 204 by collecting raw data measurements that may be used to derive a set of D data signals $s = (s_1,...,s_D)$ as indicated at block 208. Embodiments are not constrained by the type of measurements that are made at block 204 and may generally operate on any data set. For example, data signals can be retrieved from memory (e.g., storage device 108) and/or can be provided from a sensor or other input device (e.g., sensors 130). A set of K current or future outcomes $\vec{o} = (o_1,...,o_K)$ is hypothesized at block 212. The method autonomously generates a predictive model M that relates the derived data signals $\vec{s}$ with the outcomes $\vec{o}$. As used herein, "autonomous" means "without human intervention."

As indicated at block 216, this is achieved by identifying the most predictive set of signals $S_k$, where $S_k$ contains at least some (and perhaps all) of the derived signals $s_1$, ..., $s_D$ for each outcome $o_k$, where $k \in \{1,..., K\}$. A probabilistic predictive model $\hat{o}_k = M_k(S_k)$ is learned at block 220, where $\hat{o}_k$ is the prediction of outcome $o_k$ derived from the model $M_k$ that uses as inputs values obtained from the set of signals $S_k$, for all $k \in \{1,..., K\}$. Method 200 can learn the predictive models $\hat{o}_k = M_k(S_k)$ incrementally from data that contains example values of signals $s_1$, ..., $s_D$ and the corresponding outcomes $o_1$, ..., $o_K$. As the data become available, the method loops so that the data are added incrementally to the model for the same or different sets of signals $S_k$ (for all $k \in \{1,..., K\}$).

While the above outlines the general characteristics of the methods, additional features are noted. A linear model framework may be used to identify predictive variables for each new increment of data. In a specific embodiment, given a finite set of data of signals and outcomes $\{(\vec{s}_1,\vec{o}_1),(\vec{s}_2,\vec{o}_2),...\}$, a linear model may be constructed that has the form, for all $k \in \{1,..., K\}$:

$$\hat{o}_k = f_k(a_0 + \sum_{i=1}^{d} a_i s_i)$$

where $f_k$ is any mapping from one input to one output, and $a_0$, $a_1$, ...$a_d$ are the linear model coefficients. The framework used to derive the linear model coefficients may estimate which signals $s_1$, $s_2$, ..., $s_d$ are not predictive and accordingly sets the corresponding coefficients $a_1$, $a_2$, ..., $a_d$ to zero. Using only the predictive variables, the model builds a predictive density model of the data, $\{(\vec{s}_1,\vec{o}_1), (\vec{s}_2,\vec{o}_2),...\}$. For each new increment of data, a new predictive density models can be constructed.

In some embodiments, a prediction system can be implemented that can predict future results from previously analyzed data using a predictive model and/or modify the predictive model when data does not fit the predictive model. In some embodiments, the prediction system can make predictions and/or adapt the predictive model in real-time. Moreover, in some embodiments, a prediction system can use large data sets to not only create the predictive model, but also predict future results as well as adapt the predictive model.

In some embodiments, a self-learning, prediction device can include a data input, a processor and an output. Memory can include application software that when executed can direct the processor to make a prediction from input data based on a predictive model. Any type of predictive model can be used that operates on any type of data. In some embodiments, the predictive model can be implemented for a specific type of data. In some embodiments, when data is received the predictive model can determine whether it understands the data according to the predictive model. If the data is understood, a prediction is made and the appropriate output provided based on the predictive model. If the data is not understood when received, then the data can be added to the predictive model to modify the model. In some embodiments, the device can wait to determine the result of the specified data and can then modify the predictive model accordingly. In some embodiments, if the data is understood by the predictive model and the output generated using the predictive model is not accurate, then the data and the outcome can be used to modify the predictive model. In some embodiments, modification of the predictive model can occur in real-time.

In some embodiments, a predictive model can be used for medical data, robotics data, weather data, financial market data, traffic pattern data, etc.

**General Physiological Predictions**

Embodiments of the present invention provide for real time prediction of physiological conditions using various physiological data. Physiological data can be received (e.g., input) from a physiological sensor that is measuring a physiological state of a patient. Physiological feature data can then be derived from the physiological data. For example, a Finometer (physiological sensor) can be used to measure the blood pressure of a patient and provide blood pressure data (physiological data). From the blood pressure data blood volume data (physiological feature data) can be derived. Various other physiological feature data can be derived from the physiological data. From the physiological feature data a prediction can be made about a physiological threshold where patient state is reached (e.g., trauma or shock). The prediction can be based on a large data set of physiological feature data. Moreover, the prediction can use any type of predictive algorithm and/or can be self learning. In some embodiments, a user interface can provide the physiological feature data along with the predicted threshold. Such a user interface can allow a user to determine whether the physiological feature data is converging and/or diverging with the threshold data.

**Patient Blood Volume**

Hemorrhage is a problem that surgeons commonly face. It accounts for 40% of all trauma deaths and it is the most frequent cause of preventable death after severe injury. Tissue trauma can cause hemorrhage, which initiates coagulation and fibrinolysis. Shock is a primary driver of early coagulopathy. In fact, several groups have noted a linear correlation between the severity of tissue hypoperfusion and the degree of admission coagulopathy as measured by the prothrombin time (PT) and partial thromboplastin time (PTT). Recent evidence suggests that the early identification of hemorrhage, together with treatment directed at the prevention of hypotension, correction of post-injury coagulopathy and stopping the bleeding can lead to dramatic reductions in the morbidity and mortality of severely injured patients.

The problem is that humans cannot detect early signs of hemorrhage by looking at a patient's vital signs. Standard vital signs, such as heart rate, blood pressure, and arterial oxygen saturation appear to a human to change very little until a patient has lost about 30% of their total blood volume. Late detection of acute blood loss is associated with inadequate fluid resuscitation. Inadequate resuscitation poses the risk of hypotension, end organ damage and worsening coagulopathy. Conversely, aggressive fluid resuscitation may dislodge clots from vascular injuries, resulting in further blood loss, hemodilution and possibly death.

In some embodiments, the predictive model can be used to predict blood loss volume. Such embodiments can be used to detect the early signs of hemorrhage. In order to make bleeding related treatment decisions, embodiments described herein can provide information about how much blood a patient has lost. In some embodiments, the self-learning predictive model described above can be implemented to measure blood loss volume. Such predictions can be useful, for example, to aide in determining whether a wounded soldier is safe to remove from the battlefield without an IV, or whether the wounded soldier should receive intravenous fluid(s) (such as blood or saline) and/or medication prior to and during extraction.

Embodiments of the invention can also predict when an individual patient will experience CV collapse. This can be important, because individual patients experience hemodynamic decompensation at differing volumes of blood loss. On the battlefield, medics must also establish a triage order and evacuate potential survivors at greatest risk for CV collapse first. In civilian settings, paramedics and emergency medicine technicians (EMTs) must respond similarly to quickly determine who should be transported first and where. Some embodiments of the invention can provide objective, real time guidance during this critical decision-making process.

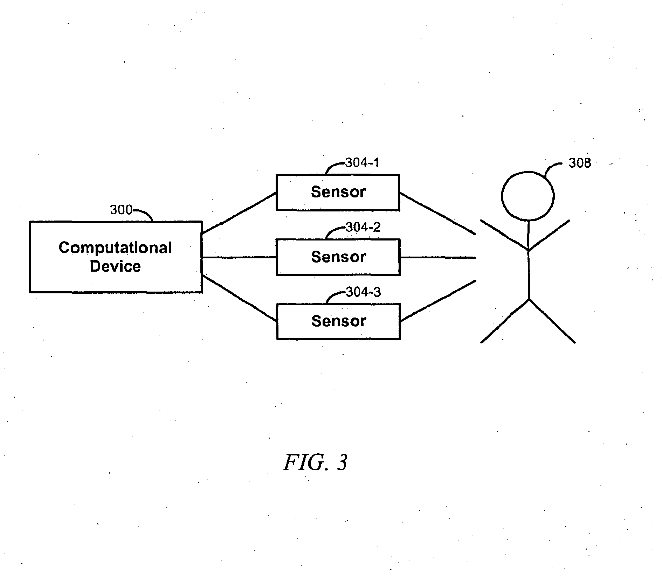

A general overview of a structure used in embodiments of the invention is provided with FIG. 3, which shows schematically that a subject 308 may have a one or more physiological sensors 304 (e.g., sensors 108 in FIG. 1) configured to read physiological data from the subject 308. The sensors 304 are provided in communication with a computational device 300 (e.g., the computational device shown in FIG. 1) configured to implement methods of the invention in predicting blood-loss volume from the subject 308. Input from sensors 304 can be the data signals and/or outcomes that are applied to the predictive model described above.

There are numerous sensors 304 that may be used in different embodiments, some of which are described herein. For example, an electrocardiograph may be used to measure the heart's electrical activity using electrodes placed specifically on the subject's 308 body. A pulse oximeter or a photoplethysmograph can be used, for example, to measure ratios of deoxygenated and oxygenated blood. As another example, a Finometer, impedance cardiography, and Finopres systems can be used to measure systolic blood pressure, diastolic blood pressure, mean arterial blood pressure, pulse pressure variability, stroke volume, cardiac output, cardiac index, and/or systolic blood pressure variability (mmHg). In another example, an infrared spectrometer can be used to measure tissue oxygenation. As another example, a transcranial Doppler system can be used to measure blood flow velocities in intracranial blood vessels. As another example, a capnogram can be used to monitor the inhaled and exhaled concentration or partial pressure of carbon dioxide ($CO_2$). As yet another example, an impedance cardiograph can be used to measure stroke volume.

While the following describes the use of a few specific sensors, this disclosure can be extended to data collected using other measurement devices, such as those described above. The output of an electrocardiograph describes cardiac muscle activity through voltages along different directions between electrode pairs. The typical electrocardiograph waveform is described as a P wave, a *QRS* complex, and a *T* wave. Heart rate can be extracted from the waveform and considerable attention has been given to heart rate variability for evaluating autonomic dysfunction and its correlation to events such as increased intracranial pressure and death due to traumatic injury. The performance of heart rate variability for predicting traumatic head injury is improved by considering factors such as heart rate, blood pressure, sedation, age, and gender. There are various algorithmic definitions for computing heart rate variability from R-R intervals, which appear to perform equivalently as long as they are calculated over extended intervals, such as over five minutes or more.

Pulse oximeters and photoplethysmographs may also be used. In their basic form, pulse oximeters use the differing properties of deoxygenated and oxygenated hemoglobin for absorbing red and infrared light. Red and infrared LEDs shine through a relatively translucent site such as the earlobe or finger and a photodetector on the other side receives the light that passes through. The observed values are used to compute the ratio of red to infrared intensity, which can be used to look up the subject's saturation of peripheral oxygen level from precomputed tables. As the heart beats, blood pulses through the arteries in the measurement location, causing more light to be absorbed, thus yielding a waveform of light signals over time. This photoplethysmograph ("PPG") can be used to determine heart rate, but also analyzed in its own right. Subtracting the trough DC values, which represent constant light absorbers, what remains are the absorption properties for the varying AC component, which is arterial blood. Advances in technology have seen more light wavelengths used to distinguish oxygen ($O_2$) and carbon dioxide ($CO_2$), thus making these systems more reliable.

Use of the raw PPG signal has been shown to be correlated to systolic pressure variation ("SPV"), which in turn is correlated with hypovolemia. A comparison of the correlation of ear and finger pulse oximeter waveforms to systolic blood pressurE ("SBP") has evaluated pulse amplitude, width, and area under the curve as extracted features. Metrics on the envelope of the PPG waveform have been used to reliably detect blood sequestration of more than one liter induced by LBNP. A linear predictor for cardiac output ("CO") has been constructed based on heart rate and features extracted from the ear PPG waveform.

The perfusion index ("PI") expresses the varying versus stationary components of infrared light in the PPG as a percentage:

$$PI = \frac{AC_{IR}}{DC_{IR}} \times 100\%.$$

The correlation of PI and core-to-toe temperature difference has been shown for critically ill patients.

The Pleth Variability Index ("PVI") describes changes in PI over at least one respiratory cycle:

$$PVI = \frac{PI_{\max R} - PI_{\min R}}{PI_{\max R}} \times 100\%.$$

It has been demonstrated that PVI can predict fluid responsiveness in anaesthetized and ventilated subjects. It has also been demonstrated that PPG variation, pulse pressure variation ("PPV"), and systolic pressure variation ("SPV") are well correlated to gradual autodonation to a reduction of 20% in systolic blood pressure.

Blood pressure and volume measurements may use the Finopres system, which may in turn use a volume clamp mechanism to measure the finger arterial pressure waveform as well as estimating parameters such as cardiac output ("CO") and stroke volume ("SV"). The mechanism combines an infrared plethysmograph to determine baseline unloaded artery diameter and monitor blood volume, and an inflatable finger cuff that is controlled to maintain baseline diameter. Variation in cuff pressure provides an indirect way of measuring intra-arterial pressure.

Similar parameters can be obtained using impedance cardiography ("ICG"), which measures volumetric changes due to the cardiac cycle by observing changes in thoracic impedance. Current is passed through the chest between sensors, traveling through the aorta as the path of least resistance. As blood velocity and volume change in the aorta, corresponding changes in impedance are recorded as a continuous waveform, from which hemodynamic parameters such as CO and SV can be computed.

Many standard hemodynamic parameters intended to capture the behavior of the cardiac cycle are derived from blood pressure and heart-rate measurements. For example, arterial blood pressure ("ABP") is the pressure in the arteries, which varies through the systolic and diastolic phases of the cardiac cycle. Systolic blood pressure ("SBP") is the maximum ABP as the left ventricle contracts. It can be extracted as the peak values of the raw Finopres ABP waveform. Diastolic blood pressure ("DBP") is the ABP when the heart is at rest. It can be measured from the troughs of the ABP waveform.

Mean arterial pressure ("MAP") describes the mean arterial blood pressure over a cardiac cycle,

$$MAP = (CO \times SVR) + CVP,$$

where CO is the cardiac output, SVR is the systemic vascular resistance, and CVP is the central venous pressure. The MAP can be approximated using more accessible parameters as

$$MAP \cong DBP + \tfrac{1}{8}(SBP - DBP).$$

Systolic pressure variability SPV attempts to measure the change or variability in SBP over a respiration cycle. In general, it is the difference (or % change) between minimum and maximum SBP,

$$SPV = SBP_{\max R} - SBP_{\min R}.$$

Distinctions are also frequently made between delta up (dUp) and delta down (dDown) components. Correlations between SPV and dDown have been examined for hemorrhage and volume replacement, finding that they follow intravascular volume for mechanically ventilated patients. One conclusion has been drawn that dDown is an effective indicator of CO response to volume replacement for mechanically ventilated septic shock patients. In some embodiments, SPV and dDown are calculated as percentages of SBP in the case of hypotension.

Pulse pressure ("PP") is the beat-to-beat change in blood pressure:

$$PP = SBP - DBP.$$

Pulse pressure variability ("PPV") is also computed using minimum and maximum PP over the respiratory cycle:

$$PPV = PP_{\max R} - PP_{\min R}.$$

It has been shown that higher PPV percentages indicate which subjects in septic shock respond to fluids and also demonstrated a correlation between PPV and cardiac index. PPV can be an effective measure for fluid management.

Stroke volume ("SV"), or volume of blood pumped by the left ventricle in a single contraction, is the difference between the amount of blood in the ventricle at the end of the diastolic phase minus the blood remaining after the heart beat:

$$SV = (\text{end diastolic volume}) - (\text{end systolic volume}).$$

Since these constituent parameters are difficult to measure, SV is generally estimated from the ABP waveform. It has been shown that SV and PP derived from finometer BP estimates are correlated with blood loss.

Cardiac output ("CO") is the volume of blood pumped per unit time:

$$CO = SV \times HR.$$

Cardiac index ("CI") relates the performance of the heart to the size of the subject using body surface area ("BSA"):

$$CI = \frac{CO}{BSA}.$$

BSA can be estimated using height and mass of the individual, and it has been found that CI and mixed venous oxygen saturation show a linear relationship to blood loss.

In other embodiments, near-infrared spectroscopy is used for measuring tissue oxygenation. In such embodiments, near-infrared light is shone on the body and deeply penetrates skin, fat, and other layers where it is either scattered or absorbed. As with pulse oximeters, the differing absorption characteristics of oxyhemoglobin ($O_2Hb$) and deoxyhemoglobin (HHb) are used to calculate concentrations based on light received by a detector. Other parameters such as pH and hematocrit can also be extracted from the spectra. This process has been modified to compensate for the interference of skin and fat layers to better measure muscle oxygen saturation ($SmO_2$). Near-infrared spectroscopy measurements of $SmO_2$ and pH have been tested as indicators of hemodynamic instability with subjects undergoing LBNP, with the conclusion that $SmO_2$ is an early indicator of vasoconstriction and impending measurements of $SmO_2$ and muscle oxygen tension ($PmO_2$) to $StO_2$ measured at the thenar eminence with a commercial device. Spectroscopic observations of $PmO_2$ and $SmO_2$ are thus early indicators of hemodynamic decompensation due to LBNP, while thenar

$StO_2$ did not change through the test.

Other noninvasive sensors, although less well investigated for monitoring hemorrhage, offer different system measurements that may contribute to the prediction system. Transcranial Doppler uses sound waves in the form of a pulsed Doppler probe to measure blood flow velocities in cerebral blood vessels (cerebral blood flow CBF). It poses challenges in determining recording locations with a clear path to the vessels of interest. CBF velocities have been used as an indicator for dynamic cerebral autoregulation under hypervolemia with hemodilution.

The respiration cycle is intimately related to the cardiac cycle and may offer relevant measurements. Capnography measures the concentration of carbon dioxide ($CO_2$) in respiratory gases and is an indirect measure of the $CO_2$ in arterial blood. Infrared light is passed through the gas sample, where $CO_2$ absorbs it and a detector on the other side observes this decrease in light. End tidal $CO_2$ (EtCO2), or the $CO_2$ concentration at the end of exhalation, has been determined to have a logarithmic relationship to cardiac output. It has also been found that $EtCO_2$ tracks SV in an LBNP model at progressive levels of central hypovolemia, but that the decreases are small relative to baseline measurements for subjects.

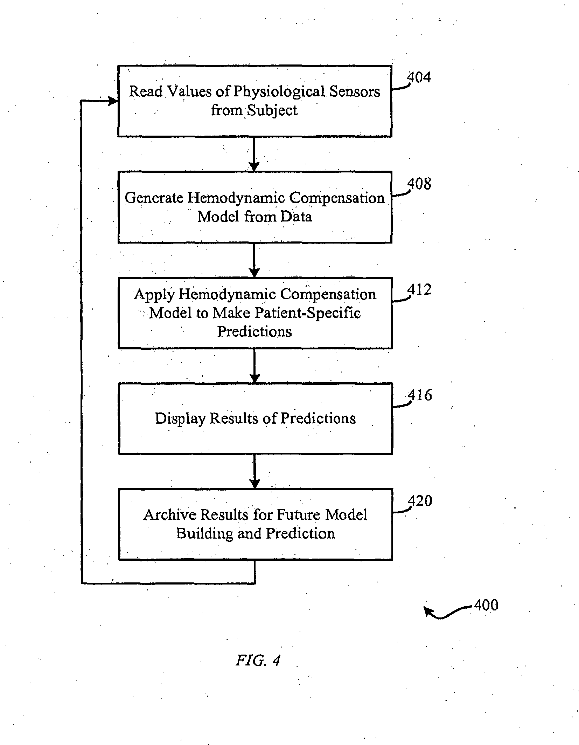

Thus, in some embodiments, a computational method for predicting the blood loss volume at which a patient will experience hemodynamic decompensation can be characterized by generating a predictive model that includes data signals $\bar{s} = (s_1,...,s_D)$ that result in outcomes $\bar{o} = (o_1,o_2)$ that ends or does not end in hemodynamic compensation. FIG. 4 shows a flowchart of a method 400 for making predictions about hemodynamic decompensation from physiological sensors. At block 404 physiological data signals can be generated and/or returned from any of the physiological sensors described above or any other physiological sensor attached with a patient. At block 408, a computational device (e.g., computational device 100 in FIG. 1) can read values from the physiological sensors can generate hemodynamic compensation models from data (e.g., $\hat{o}_k = f_k \left( a_0 + \sum_{i=1}^{d} a_i s_i \right)$). At block 412 patient specific predictions based on the hemodynamic compensation models can be made from new data signals. At block 416 the predictions can be provided to a medical practitioner, who may provide semantic (machine readable) text to the predictive model, thus augmenting the result. At block 420, the results can be saved for future model building and or predictions.

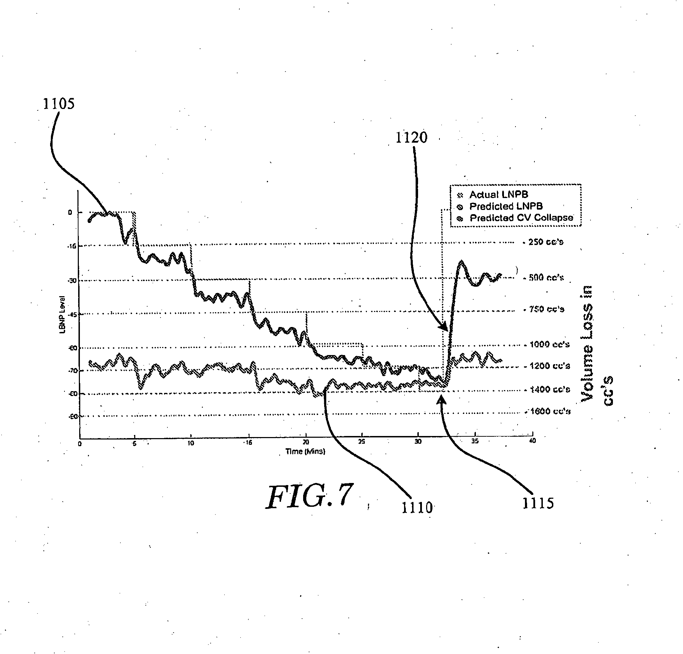

In some embodiments, a computational device (e.g., computational device 100) can simultaneously predict: 1) blood loss volume and 2) individual specific blood loss volume for CV collapse. In some embodiments, the computational device can simultaneously graph predicted blood loss volume 1105 with predicted, individual specific blood loss volume for CV collapse to occur 1110, as shown in FIG. 7. In some embodiments, the computational device can analyze noninvasively measured blood pressure (e.g., using a Finopres or other device coupled with sensor interface 130). The blood pressure data can then be converted to predicted volume of acute blood loss, as described above. The device can also predict the level of blood volume loss that will lead to CV collapse 1110. The estimated blood volume loss 1105 and the predicted point where CV collapse occurs 1115 can be provided on a single graph as shown in FIG. 7. It should be noted that this graph also provides the true blood volume loss and the true point of CV collapse 1115. Such a graph can allow both experienced and inexperienced medical personnel the ability to quickly assess how much blood a patient has lost and estimate how much and what type of fluid should be given and/or when CV collapse will likely occur. CV collapse will occur at the point where predicted blood volume loss 1105 and predicted, individual specific volume of blood loss for CV collapse 1110 converge at point 1115. Such data can help military medics as well as civilian paramedics determine who should be attended to first, whether to begin IV fluids or blood, how much fluid to give and at what rate, and when to stop giving fluids, etc.

In some embodiments, a computational device (e.g., computational device 130 in FIG. 1) can automatically determine that type of device coupled with the computational device. In some embodiments, the computational device can make such a determination from the sensor interface or based on the connector used to couple the sensor. In some embodiments, a processor can determine the data type based on any number of parameters associated with the data such as frequency, amplitude, current, digital signals, etc. In some embodiments, sensors types can vary based on the environment of the sensor. Once it is determined what type of sensor that has been coupled with the computational device, the processor can determine the proper predictive and/or self learning algorithm to use. For example, a number of predictive and/or self learning algorithms can be stored in memory and associated with a sensor and/or sensor type. One of the predictive and/or self learning algorithms can be executed based on the type sensor coupled with the sensor interface. In some embodiments, the computational unit can ensure that prediction or self learning only occurs when the sensors are properly applied to the patient. The processor can also determine the best sensor from a group of sensors based on signal quality. In some embodiments, a predictive model can be chosen from memory based on the sensor, sensor type, prediction quality, and prediction timeframe.

In some embodiments, a device can implement embodiments of the present invention for monitoring fluid levels in a patient during the delivery of intravenous fluids. As a patient is being treated with IV fluids, the device can provide medical personal with real-time information on the effectiveness of IV fluid therapy as shown in FIG. 7. If the 1105 and 1110 waveforms continue to converge, bleeding is ongoing. If the 1105 waveform flattens, IV fluid therapy is just keeping up with blood loss. If 1105 and 1110 waveforms are diverging 1120, then the provider knows, in real-time, that the rate and amount of IV fluid resuscitation is benefiting the patient. This embodiment can mitigate the guess work inherent in the delivery of IV fluids to a patient. I can provide real-time information to a practitioner on the effectiveness of IV fluid therapy, by indicating where one is and where one is going in the fluid resuscitation process.

**Noninvasive Prediction of Intracranial Pressure and Cerebral Perfusion Pressure**

Embodiments of the invention provide a number of methods and systems related to monitoring and treating various cerebral parameters. According to some embodiments of the invention, hemodynamic and/or cerebral parameters can be diligently recorded and time-synchronized. Machine learning techniques and/or predictive models can be used with this data to determine whether there are undiscovered correlations between central and cerebral physiological variables, and such correlations may be used to diagnose, trend, and predict nearly instantaneous changes in intracranial (ICP) and cerebral perfusion pressures. These hemodynamic and/or cerebral parameters can include electrocardiograph measurements, arterial blood pressures, venous pressures, carotid blood flow, intrathoracic pressures, heart rate, cardiac output, intracranial pressures, end tidal carbon dioxide values, and blood gases.

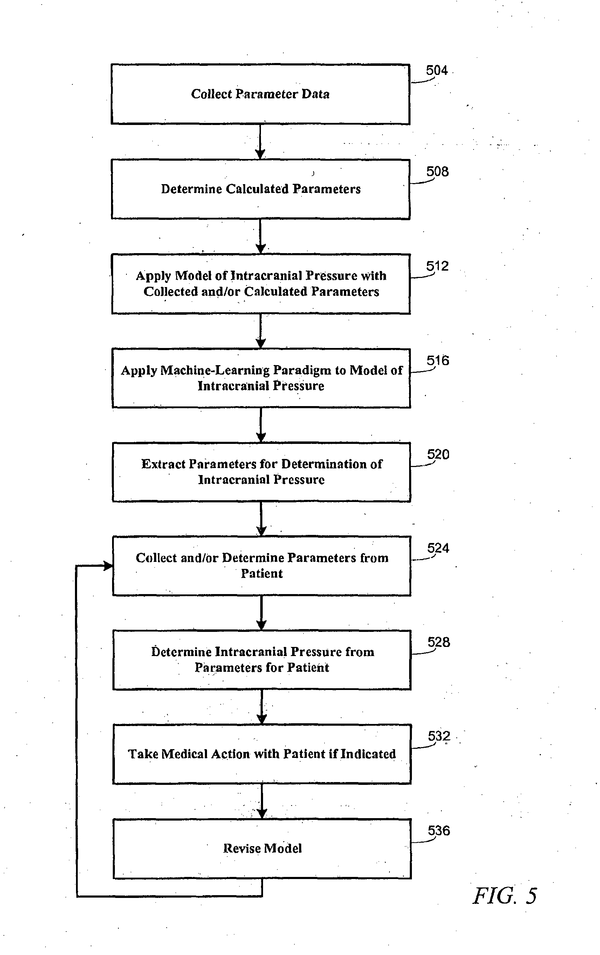

A general overview of how embodiments of the invention may be implemented is illustrated with the flow diagram of FIG. 5. In this diagram, parameter data are initially collected from a set of subjects at block 504 and may include both parameters that are collected noninvasively and invasively. Examples of noninvasively collected parameters can include heart rate, pulse oximetry and transcranial Doppler data, among other potential parameters; examples of invasively collected parameters can include systolic blood pressure and diastolic blood pressure, among others (e.g., those described above). As indicated at block 508, some parameters may be calculated, such as mean arterial pressure, cardiac output, and total peripheral resistance, among others.

In addition to these parameters, the intracranial pressure and/or the cerebral perfusion pressure may be measured and calculated so that a model of intracranial pressure may be applied at block 512 to relate such values with the various parameters obtained at blocks 504 and 508. A machine-learning paradigm (e.g., the predictive model described above) can be applied at block 516 to enable the extraction of those parameters that are most relevant to determining the intracranial pressure and/or the cerebral perfusion pressure; the model may then be tailored for prediction of those quantities at block 520.

The resultant model may then be used diagnostically as indicated in the drawing. For instance, the relevant parameters determined at block 520 may be collected at block 524 for a patient presented for diagnosis and the intracranial pressure and/or the cerebral perfusion pressure determined at block 528 by application of the model. If the determined pressure is outside of an acceptable range, medical action may be taken at block 532. In some embodiments, it can be possible for revisions to the model to be made at block 536, particularly after treatment of the patient, in order to improve the value and application of the model.

Evaluation of the model may be made in any of several different ways. For example, a mean square difference of the intracranial pressure predicted by the model and the true estimated intracranial pressure may be calculated. Similarly, mean square difference between the predicted cerebral perfusion pressure and the true estimated cerebral perfusion pressure may be calculated. When a change in intracranial pressure is detected, the time taken for the model to respond to this change in the predicted intracranial pressure or to the predicted cerebral perfusion pressure may be relevant in evaluating the model. In addition, detection of a change in intracranial pressure may be used to calculate the time taken for carotid artery blood flow to diminish and to compare this with the time taken for the model to respond to such a change.

Various studies testing embodiments of the method have enabled the prediction of ICP using hemodynamic measures such as heart rate variability and central hemodynamic pressure. The ability to predict ICP directly from these central hemodynamic parameters stems from the experimentally proven ability to predict blood volume loss and CV collapse onset, using only cranial measures of blood flow derived from intracranial Doppler signals.

Management of traumatic brain injury may include therapies and diagnostic techniques that optimize and monitor cerebral metabolism and function by minimizing global cerebral ischemia. Such therapies may be included in algorithm modifications to allow noninvasive tracking of cerebral pressures.

The machine-learning paradigm accordingly permits the establishment of models that relate such parameters as described above to the intracranial and cerebral perfusion pressures. In particular, it enables the otherwise invasive intracranial and cerebral perfusion pressures to be determined through measurement of noninvasive parameters.

**Noninvasive Prediction of Central Blood Volume Loss**

In further embodiments, lower-body negative pressure ("LBNP") can be used to simulate loss of central blood volume in humans. Such a model provides a method for investigating physiological signals under conditions of controlled, experimentally induced hypovolemic hypotension in otherwise healthy humans. In one set of studies, each subject was placed in an LBNP chamber and connected to a variety of noninvasive monitoring devices. Baseline measurements were made. Subjects were exposed to progressively greater amounts of LBNP to the point of cardiovascular collapse. At that point, the LBNP was released and central volume returned to normal. The experiments lasted between 25 and 50 minutes and were dependent on the level of LBNP at which the subject exhibited cardiovascular collapse. Each LBNP level equates to about 250 cc's of blood loss.

The inventors used the method described above to derive a machine-learning paradigm that is capable of the following in real time: (1) detecting early, primary signs of LBNP, which equate to acute blood loss; (2) estimating the rate and volume of blood loss in a bleeding patient to guide resuscitation therapy; and (3) predicting a timeframe for when a bleeding patient will progress to cardiovascular collapse. The method uses hemodynamic features as inputs derived from commercially available physiological sensors, i.e. heart rate, blood pressure and RR interval from the electrocardiograph. The sample size was 64 heart beats. For this particular embodiment, the method is about 96.5% accurate in predicting the presence of active bleeding; is about 96% accurate in identifying the level of bleeding to within 250 cc's; is about 85% accurate for predicting individual specific LBNP level that a subject will experience cardiovascular collapse. Further training of the algorithm with data from 104 LBNP subjects shows greater than 95% prediction accuracy for both LNPB level and individual specific CV collapse levels.

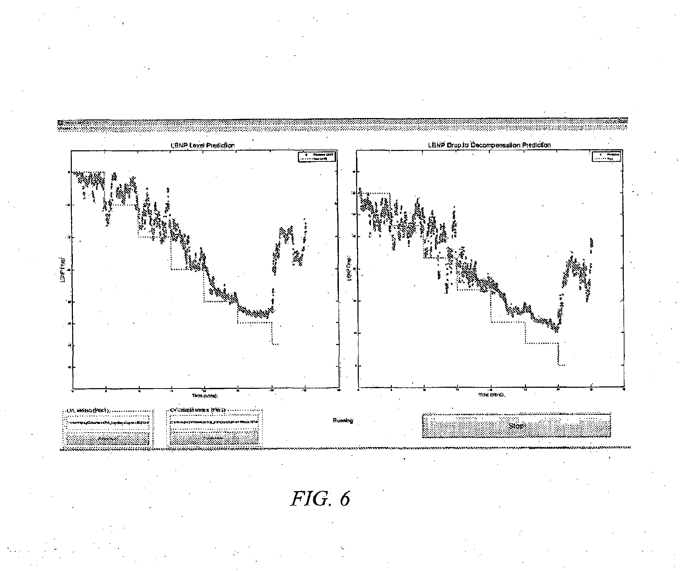

FIG. 6 shows screen shots from a device tested during a live LBNP experiment. The solid lines indicate the true LBNP level and the dots indicate predictions. The left plot shows the LBNP level, while the right plot shows the predicted drop in LBNP level needed for the subject to experience hemodynamic decompensation (CV collapse). Both predictions yielded a correlation of 0.95. Note that both sets of predictions were made in real-time, while the experiments were taking place.

**Other Healthcare Applications**

Foreseeing the clinical course of a patient whose physiology is possibly complex and constantly changing due to injury, patient disease and/or our efforts to stabilize and correct the underlying disease process depends on a practitioner's ability to identify, understand and continuously monitor a range of clinical features. Practitioners cannot, of course, physically reside at a patient's bedside at all times. Nor can they rapidly abstract, discern and respond to the many unique and subtle features that are characteristic of normal and abnormal physiological signals. Embodiments of the invention can apply a new polynomial Mahalanobis distance metric for use in classifying continuous physiological data (e.g., any waveform data), to enable active, long term learning from extremely large continually changing physiological datasets. The application of such embodiments to human vital sign data has led to the discovery of several previously hidden hemodynamic relationships that are predictive of acute blood loss and individual specific risk for cardiovascular collapse. Implementation of embodiments of the invention have broad applicability in many areas of medicine and surgery. It is especially applicable to the care of severely injured patients, whose physiology is acute, complex, constantly changing and human interpretation is required on an ongoing basis.

Embodiments of the invention incorporate dynamic, multi-objective optimization schemes. Such schemes can become increasingly more complex as greater amounts of high fidelity clinical data is captured and becomes available for analysis. Dynamic multi-objective optimization schemes can enable the development of predictive models using real-time physiological data, while autonomously controlling the management of competing therapies. An example is IV fluid management for an injured soldier with a traumatic brain injury *and* an exsanguinating solid organ injury. IV fluid therapy in this type of setting must be provided at a rate that will optimize systemic and cerebral perfusion, avoid re-bleeding and maintain the patient until bleeding can be controlled. Competing injuries add complexity to any fluid resuscitation strategy and the invention described herein solves this problem.

In some embodiments, the inputs to a predictive device can include non-invasively measured physiological signals, derived from existing products used in medical facilities. In some instances, the device comprises a laptop computer (e.g., as schematically shown in FIG. 1) that runs a codified method for hemodynamic monitoring with accuracies as good as or better than conventional methods. Such a device can interface to a variety of standard medical sensors, including an EKG and/or a non-invasive Finopres blood pressure monitor. Other embodiments can include devices that detect when one or more sensors are incorrectly attached to a patient. Still other embodiments include devices that automatically choose the most accurate and relevant set of models, based on: available sensors and how long the patient has been monitored.

Some methods and devices of the invention provide an intuitive user interface to allow medical professionals to interact with the device. In some embodiments, the user interface can allow the user to specify which sensors are available, which can then define which model to use. The user interface can also allow the device to intuitively interact with the medical professional to ensure correct sensor functioning and/or allow the medical professional to enter patient specific clinical information such as gender, weight, age, historical information, physical exam findings, various forms of treatment and information on the clinical response to treatment. In some embodiments, this clinical information can be retrieved from various data sources include computer hard drivers, network drives, etc. In some embodiments this information can be retrieved from central servers that have historical health and patient information stored thereon.

These results indicate that methods of the invention for analyzing noninvasive hemodynamic parameters is not only fast and accurate, but a viable platform for a device that could provide medical personnel with early, reliable and critically important information on blood loss, injury severity and the time to act.

Devices and methods of the invention can be seamlessly integrated into existing hospital and pre-hospital care settings because: they can be applied in parallel with existing physiological monitors, medical personnel need not change standard procedures, the method alone could be licensed to device manufactures, to enable existing, in-hospital monitors to become "smart" monitors.

Some devices of the invention utilize advanced hemodynamic measures, derived from traditional monitoring devices (blood pressure, EKG, etc). Some devices of the invention can be used to collect non-invasive data. A large amount of data can be collected from individual patients, requiring relatively few subjects for verification. Verification can be done in a short period of time, as no lengthy experimental procedures and no blood work are required. Some devices of the invention have low computational requirements (i.e. they can effectively run on inexpensive processors and laptop computers).

Methods and devices of the invention can save lives by providing early, critical information on acute blood loss, injury severity, and resuscitation effectiveness. This invention will be of great commercial interest to all branches of the U.S armed services, trauma and non-trauma surgeons, anesthesiologists and critical care physicians worldwide. It is equally useful during the management of trauma and non-trauma patients, who are experiencing or are at risk volume loss, whether it be due to the acute loss of blood, dehydration and/or myocardial dysfunction.

## Conclusion

Embodiments of the invention can be adapted to any condition for which there exists subject data. In the medical arena this type of data will increase exponentially in the coming years, as physiological data from individual illness events becomes incorporated into each patient's electronic medical record. The matching of physiological patient data with semantically driven medical records containing various diagnoses, the timing of therapy and response to treatment, will allow methods and devices of the invention to gain insight into the practice of medicine and expected outcomes. For example, self-learning predictive systems may provide predictions based not only on real-time physiological measurements, but also on a patient's medical history such as age, diet, previous diagnoses, exercise routine, smoking habits, caffeine intake, alcohol consumption, travel history, various medical risk factors, familial history, allergies, pharmaceutical intake, weight, physical exam findings, practitioner impressions and treatment effects, etc. Moreover, multiple physiological measurements can be used to make predictions and/of for self learning.

Examples of medical and surgical conditions that could be analyzed and potentially linked and evaluated in real-time using aspects of the various embodiments include: 1) closed head injury monitoring and management, including cEEG; 2) differentiation of shock states; 3) resuscitation monitoring and management; 4) asthma, pneumonia and other respiratory diseases; 5) diabetes monitoring and prevention of diabetic ketoacidosis; 6) myocardial ischemia and infarction; 7) stroke; 8) congestive heart failure; 9) intra-operative monitoring, including depth of anesthesia; 10) pain control monitoring and management; 12) post-operative monitoring; 13) sleep apnea monitoring; 14) rehabilitation monitoring, including gait, stability and range of motion; cognitive function; activities of daily living; 15) progressive neurological disorders, e.g. Alzheimer's disease, multiple sclerosis, epilepsy, etc.; and 16) therapeutic oncology, to name a few.

## Claims

1. A method for predicting physiological phenomena, the method comprising:

   receiving (524) real-time physiological data from a physiological sensor (130, 204-1, 304-2, 304-3) that is measuring a physiological characteristic of a patient;
   deriving (412, 528) physiological feature data from the physiological data;
   **characterized by**
   determining, from a predictive model, a physiological threshold from the physiological feature data and from historical data, wherein the physiological threshold corresponds to a point such that when the physiological feature data reaches the physiological threshold abnormal physiology is deemed to be present, the predictive model comprising a set of probabilistic predictive model $\hat{o}_k = M_k(S_k)$, where $\hat{o}_k$ is a prediction of outcome $o_k$ derived from the model $M_k$ that uses as inputs values obtained from a set of signals $S_k$ derived using the physiological data from the physiological sensor (130, 204-1, 304-2, 304-3); and
   providing (416) at a user interface the relationship between the physiological threshold and the physiological feature data as physiological feature data is derived from the physiological data;

wherein the predictive model is determined using the steps of:
identifying (216) the set of signals $S_k$ as a most-predictive set of signals $S_k$ out of a set of signals $s_1, s_2, ..., s_D$ for each of one or more outcomes $o_k$;
autonomously learning (220) the set of probabilistic predictive models $\hat{o}_k = M_k(S_k)$, where $\hat{o}_k$ is a prediction of outcome $o_k$ derived from the model $M_k$ that uses as inputs values obtained from the set of signals $S_k$;
repeating the step of autonomously learning (220) incrementally from data that contains examples of values of signals $s_1; s_2, ..., s_D$ and corresponding outcomes $o_1, o_2, ..., o_K$; and
building (214) the predictive model from the signals and corresponding outcomes.

2. The method of claim 1 wherein autonomously learning (220) the set of probabilistic predictive models comprises using a linear model framework to identify predictive variables for each increment of data.

3. The method of claim 2 wherein the linear model framework is constructed with the form $\hat{o}_k = f_k\left( a_0 + \sum_{i=1}^{d} a_i s_i \right)$, where $f_k$ is a mapping function mapping one input to one output and $\alpha_0, \alpha_1, ..., \alpha_d$ are linear model coefficients.

4. The method recited in any of claims 1, 2, or 3, wherein:

the physiological threshold is volume of blood loss,
the predictive model is a hemodynamic compensation model;
determining a physiological threshold comprises applying the hemodynamic compensation model to the real-time physiological data and/or the derived physiological feature data, to predict the volume of acute blood loss from the patient; and
the hemodynamic compensation model is generated from the historical data, the historical data comprising a plurality of data values collected from physiological sensors attached to a plurality of subjects.

5. The method recited in any of claims 1, 2, or 3, wherein:

the physiological threshold is volume of acute blood loss from a patient that will cause cardiovascular collapse;
the predictive model is a hemodynamic compensation model;
determining the physiological threshold comprises applying the hemodynamic compensation model to the real-time physiological data and/or the derived physiological feature data to predict the volume of acute blood loss from the patient that will cause cardiovascular collapse; and
the hemodynamic compensation model is generated from the historical data, the historical data comprising a plurality of data values previously collected from physiological sensors attached to a plurality of subjects.

6. The method recited in any of claims 4 or 5, wherein the one or more physiological sensors comprises a sensor selected from the group consisting of a blood pressure monitor, a noninvasive blood pressure monitor, an electrocardiograph, a pulse oximeter, a transcranial Doppler sensor, an impedance cardiograph, a finometer, an infrared spectrometer, an impedance cardiograph, a capnography sensor, and a photoplethysmograph.

7. The method recited in any of claims 4 or 5, wherein the collected data values comprise data values selected from the group consisting of a perfusion index, a pleth variability index, cardiac output, heart stroke volume, arterial blood pressure, systolic blood pressure, diastolic blood pressure, mean arterial pressure, systolic pressure variability, pulse pressure, pulse pressure variability, stroke volume, cardiac index, and near-infrared spectroscopy data.

8. The method of claim 1, further comprising:

deriving second physiological feature data from the physiological data;
determining a second physiological threshold from the second physiological feature data and from historical data, wherein the second physiological threshold corresponds to a point such that when the second physiological feature data reaches the second physiological threshold a different physiological event occurs or is detected; and
providing at a user interface the relationship between the second physiological threshold and the physiological feature data as the second physiological feature data is derived.

9. The method recited in any of claims 1-8, wherein the providing includes graphing the physiological threshold and the physiological feature data as a function of time.

10. The method recited in any of claims 1-9, wherein the physiological data comprises data selected from the list consisting of blood pressure data, EEG data, heart rate data, deoxygenated blood data, oxygenated blood data, muscular activity, and oxygen inhalation.

11. The method recited in any of claims 1-10, wherein the physiological feature data comprises data selected from the list consisting of systolic blood pressure, arterial blood pressure, mean arterial blood pressure, pulse pressure variability, stroke volume, cardiac output, cardiac index, systolic pressure variability, and diastolic blood pressure.

12. The method recited in any of claims 1-11, further comprising determining a physiological response to treatment by monitoring the convergence or divergence of the physiological threshold and the physiological feature data as a function of time.

13. A system for predicting physiological phenomena, the system comprising:

    a processor (102) and a computer readable storage medium (110b), the computer readable storage medium (110b) comprising:

        instructions for receiving real-time physiological data from a physiological sensor (130) that is measuring a physiological characteristic of a patient;
        instructions for deriving physiological feature data from the physiological data;

        **characterized by**
        instructions for determining, from a predictive model, a physiological threshold from the physiological feature data and from historical data, wherein the physiological threshold corresponds to a point such that when the physiological feature data reaches the physiological threshold abnormal physiology is deemed to be present, the predictive model comprising a set of probabilistic predictive models $\hat{o}_k = M_k(S_k)$, where $\hat{o}_k$ is a prediction of outcome $o_k$ derived from the model $M_k$ that uses as inputs values obtained from a set of signals $S_k$ derived using the physiological data from the physiological sensor (130, 204-1, 304-2, 304-3), wherein the instructions for determining the physiological threshold from the physiological feature data comprise:

            instructions for identifying the set of signals $S_k$ as a most-predictive set of signals $S_k$ out of a set of signals $s_1, s_2, ..., s_D$ for each of one or more outcomes $o_k$;
            instructions for autonomously learning the set of probabilistic predictive models $\hat{o}_k = M_k(S_k)$, where $\hat{o}_k$ is a prediction of outcome $o_\kappa$ derived from the model $M_k$ that uses as inputs values obtained from the set of signals $S_k$;
            instructions for repeating the step of autonomously learning incrementally from data that contains examples of values of signals $s_1, s_2, ..., s_D$ and corresponding outcomes $o_1, o_2, ..., o_K$; and
            instructions for building the predictive model from the signals and corresponding outcomes; and

    instructions for providing at a user interface (106) the relationship between the physiological threshold and the physiological feature data as physiological feature data is derived from the physiological data.

**Patentansprüche**

1. Verfahren zur Prognose physiologischer Phänomene, umfassend:

    das Empfangen (524) physiologischer Daten in Echtzeit von einem physiologischen Sensor (130, 204-1, 304-3), der ein physiologisches Merkmal eines Patienten misst;
    das Ableiten (412, 528) physiologischer Merkmalsdaten von den physiologischen Daten;
    **gekennzeichnet durch**
    die Bestimmung, anhand eines Prognosemodells, eines physiologischen Schwellenwerts anhand der physiologischen Merkmalsdaten und anhand historischer Daten, wobei der physiologische Schwellenwert einem Punkt entspricht, sodass, wenn die physiologischen Merkmalsdaten den physiologischen Schwellenwert erreichen, angenommen wird, dass eine abnormale Physiologie vorliegt, wobei das Prognosemodell einen Satz wahrscheinlichkeitstheoretischer Prognosemodelle $\hat{o}_k = M_k(S_k)$ umfasst, wobei $\hat{o}_k$ eine Prognose des Ergebnisses $o_k$ ist, abgeleitet von dem Modell $M_k$, das als Eingaben Werte verwendet, erhalten von einem Satz von Signalen $S_k$, abgeleitet unter Verwendung der physiologischen Daten von dem physiologischen Sensor (130, 204-1, 304-2, 304-3); und

die Bereitstellung (416) an einer Benutzerschnittstelle der Beziehung zwischen dem physiologischen Schwellenwert und den physiologischen Merkmalsdaten als physiologische Merkmalsdaten, abgeleitet von den physiologischen Daten;

wobei das Prognosemodell bestimmt wird unter Verwendung der folgenden Schritte:

Identifizieren (216) des Satzes von Signalen $S_k$ als den prädiktivsten Satz von Signalen $S_k$ aus einem Satz von Signalen $s_1, s_2,..., s_D$ für jedes von einem oder mehr Ergebnissen $o_k$;

autonomes Lernen (220) des Satzes wahrscheinlichkeitstheoretischer Prognosemodelle $\hat{o}_k = M_k(S_k)$, wobei $\hat{o}_k$ eine Prognose von Ergebnis $o_k$ ist, abgeleitet von dem Modell $M_k$, das als Eingaben Werte verwendet, erhalten von einem Satz von Signalen $S_k$;

Wiederholen des Schritts des autonomen Lernens (220) inkremental anhand von Daten, die Beispiele von Werten von Signalen $s_1, s_2,..., s_D$ und entsprechenden Ergebnissen $o1, o2, ... o_K$ enthalten; und

Aufbauen (214) des Prognosemodells anhand der Signale und der entsprechenden Ergebnisse.

2.  Verfahren nach Anspruch 1, wobei das autonome Lernen (220) des Satzes wahrscheinlichkeitstheoretischer Prognosemodelle die Verwendung eines linearen Modellrahmens zur Identifizierung von Prognosevariablen für jedes Dateninkrement umfasst.

3.  Verfahren nach Anspruch 2, wobei der lineare Modellrahmen konstruiert wird mit der Formel $\hat{o}_k = f_k\left(a_0 + \sum_{i=1}^{d} a_i s_i\right)$,

    wobei $f_k$ eine Abbildungsfunktion ist, um eine Eingabe einer Ausgabe zuzuordnen, und $\alpha_0, \alpha_1, ..., \alpha_d$ lineare Modellkoeffizienten sind.

4.  Verfahren nach einem der Ansprüche 1, 2 oder 3, wobei:

    der physiologische Schwellenwert ein Volumen von Blutverlust ist,

    das Prognosemodell ein hämodynamisches Kompensationsmodell ist;

    die Bestimmung eines physiologischen Schwellenwertes die Anwendung des hämodynamischen Kompensationsmodells auf die physiologischen Echtzeit-Daten und/oder die abgeleiteten physiologischen Merkmalsdaten umfasst, um das Volumen des akuten Blutverlustes bei dem Patienten zu prognostizieren; und

    das hämodynamische Kompensationsmodell anhand der historischen Daten erzeugt wird, wobei die historischen Daten mehrere Datenwerte umfassen, erfasst von physiologischen Sensoren, angebracht bei mehreren Probanden.

5.  Verfahren nach einem der Ansprüche 1, 2 oder 3, wobei:

    der physiologische Schwellenwert ein Volumen von akutem Blutverlust bei einem Patienten ist, der zu einem Herz-Kreislauf-Versagen führen wird;

    das Prognosemodell ein hämodynamisches Kompensationsmodell ist;

    die Bestimmung des physiologischen Schwellenwertes die Anwendung des hämodynamischen Kompensationsmodells auf die physiologischen Echtzeit-Daten und/oder die abgeleiteten physiologischen Merkmalsdaten umfasst, um das Volumen von akutem Blutverlust bei dem Patienten, der zu einem Herz-Kreislauf-Versagen führen wird, zu prognostizieren; und

    das hämodynamische Kompensationsmodell anhand der historischen Daten erzeugt wird, wobei die historischen Daten mehrere Datenwerte umfassen, die zuvor von physiologischen Sensoren, angebracht bei mehreren Probanden, erfasst wurden.

6.  Verfahren nach einem der Ansprüche 4 oder 5, wobei der eine Sensor oder die mehreren Sensoren einen Sensor umfassen, ausgewählt aus der Gruppe, bestehend aus einem Blutdrucküberwachungsgerät, einem nichtinvasiven Blutdrucküberwachungsgerät, einem Elektrokardiographen, einem Pulsoximeter, einem transkraniellen Doppler-Sensor, einem Impedanzkardiographen, einem Finimeter, einem Infrarot-Spektrometer, einem Impedanzkardiographen, einem Kapnographiesensor und einem Photoplethysmographen.

7.  Verfahren nach einem der Ansprüche 4 oder 5, wobei die erfassten Datenwerte Datenwerte umfassen, ausgewählt aus der Gruppe, bestehend aus einem Perfusionsindex, einem Plethvariabilitätsindex, einem Herzauswurfvolumen, einem Herzschlagvolumen, einem arteriellen Blutdruck, einem systolischen Blutdruck, einem diastolischen Blutdruck, einem mittleren Arteriendruck, einer systolischen Druckvariabilität, einem Pulsdruck, einer Pulsdruckvariabilität, einem Schlagvolumen, einem Herzindex und Nah-Infrarot-Spektroskopiedaten.

8. Verfahren nach Anspruch 1, ferner umfassend:

die Ableitung physiologischer Merkmalsdaten anhand der physiologischen Daten;
die Bestimmung eines zweiten physiologischen Schwellenwertes anhand der zweiten physiologischen Merkmalsdaten und anhand von historischen Daten, wobei der zweite physiologische Schwellenwert einem Punkt entspricht, sodass, wenn die zweiten physiologischen Merkmalsdaten den zweiten physiologischen Schwellenwert erreichen, ein anderes physiologisches Ereignis stattfindet oder detektiert wird; und
die Bereitstellung an einer Benutzerschnittstelle der Beziehung zwischen dem zweiten physiologischen Schwellenwert und den physiologischen Merkmalsdaten bei Ableitung der zweiten physiologischen Merkmalsdaten.

9. Verfahren nach einem der Ansprüche 1-8, wobei die Bereitstellung die grafische Darstellung des physiologischen Schwellenwertes und der physiologischen Merkmalsdaten abhängig von der Zeit umfasst.

10. Verfahren nach einem der Ansprüche 1-9, wobei die physiologischen Daten Daten umfassen, ausgewählt aus der Liste, bestehend aus Blutdruckdaten, EEG-Daten, Herzfrequenzdaten, deoxygenierten Blutdaten, oxygenierten Blutdaten, Muskelaktivität und Sauerstoffeinatmung.

11. Verfahren nach einem der Ansprüche 1-10, wobei die physiologischen Merkmalsdaten Daten umfassen, ausgewählt aus der Liste, bestehend aus systolischem Blutdruck, arteriellem Blutdruck, mittlerem arteriellem Blutdruck, Pulsdruckvariabilität, Schlagvolumen, Herzauswurfvolumen, Herzindex, systolischer Druckvariabilität und diastolischem Blutdruck.

12. Verfahren nach einem der Ansprüche 1-11, ferner umfassend die Bestimmung einer physiologischen Reaktion auf eine Behandlung durch Überwachung der Konvergenz oder Divergenz des physiologischen Schwellenwertes und der physiologischen Merkmalsdaten abhängig von der Zeit.

13. System zur Prognose physiologischer Phänomene, umfassend:

einen Prozessor (102) und ein computerlesbares Speichermedium (110b), wobei das computerlesbare Speichermedium (110b) Folgendes umfasst:

Befehle zum Empfangen physiologischer Daten in Echtzeit von einem physiologischen Sensor (130), der ein physiologisches Merkmal bei einem Patienten misst;
Befehle zur Ableitung physiologischer Merkmalsdaten anhand der physiologischen Daten;
**gekennzeichnet durch**
Befehle zur Bestimmung, anhand eines Prognosemodells, eines physiologischen Schwellenwertes anhand der physiologischen Merkmalsdaten und anhand historischer Daten, wobei der physiologische Schwellenwert einem Punkt entspricht, sodass, wenn die physiologischen Merkmalsdaten den physiologischen Schwellenwert erreichen, angenommen wird, dass eine abnormale Physiologie vorliegt, wobei das Prognosemodell einen Satz wahrscheinlichkeitstheoretischer Prognosemodelle $\hat{o}_k = M_k(S_k)$ umfasst, wobei $\hat{o}_k$ eine Prognose des Ergebnisses $o_k$ ist, abgeleitet von dem Modell $M_k$, das als Eingaben Werte verwendet, erhalten von einem Satz von Signalen $S_k$, abgeleitet unter Verwendung der physiologischen Daten von dem physiologischen Sensor (130, 204-1, 304-2, 304-3), wobei die Befehle zur Bestimmung des physiologischen Schwellenwertes anhand der physiologischen Daten umfassen:

Befehle zum Identifizieren des Satzes von Signalen $S_k$ als den prädiktivsten Satz von Signalen $S_k$ aus einem Satz von Signalen $s_1, s_2,..., s_D$ für jedes von einem oder mehr Ergebnissen $o_k$;
Befehle zum autonomen Lernen des Satzes wahrscheinlichkeitstheoretischer Prognosemodelle $\hat{o}_k = M_k(S_k)$, wobei $\hat{o}_k$ eine Prognose des Ergebnisses $o_k$ ist, abgeleitet von dem Modell $M_k$, das als Eingaben Werte verwendet, erhalten von einem Satz von Signalen $S_k$;
Befehle zum Wiederholen des Schritts des autonomen Lernens inkremental anhand von Daten, die Beispiele von Werten von Signalen $s_1, s_2,..., s_D$ und entsprechenden Ergebnissen $o1, o2, ... o_K$ enthalten; und
Befehle zum Aufbauen des Prognosemodells anhand der Signale und der entsprechenden Ergebnisse; und
Befehle zum Bereitstellen, an einer Benutzerschnittstelle (106), der Beziehung zwischen dem physiologischen Schwellenwert und der physiologischen Merkmalsdaten bei Ableitung physiologischer Merkmalsdaten von den physiologischen Daten.

**Revendications**

1. Procédé de prédiction de phénomènes physiologiques, le procédé comprenant les étapes ci-dessous consistant à :

   recevoir (524) des données physiologiques en temps réel en provenance d'un capteur physiologique (130, 204-1, 304-2, 304-3) qui mesure une caractéristique physiologique d'un patient ;
   dériver (412, 528) des données de fonctions physiologiques à partir des données physiologiques ;
   **caractérisé par** les étapes ci-dessous consistant à :

   déterminer, à partir d'un modèle prédictif, un seuil physiologique à partir des données de fonctions physiologiques et de données historiques, dans lequel le seuil physiologique correspond à un point de sorte que lorsque les données de fonctions physiologiques atteignent le seuil physiologique, une anomalie physiologique est considérée comme étant présente, le modèle prédictif comprenant un ensemble de modèles prédictifs probabilistes $\hat{o}_k = M_k(S_k)$, où $\hat{o}_k$ est une prédiction de résultat $o_k$ dérivée du modèle $M_k$ qui utilise, en tant qu'entrées, des valeurs obtenues à partir d'un ensemble de signaux $S_k$ dérivé en utilisant les données physiologiques en provenance du capteur physiologique (130, 204-1, 304-2, 304-3) ; et
   fournir (416), au niveau d'une interface d'utilisateur, la relation entre le seuil physiologique et les données de fonctions physiologiques, à mesure que des données de fonctions physiologiques sont dérivées des données physiologiques ; dans lequel le modèle prédictif est déterminé en mettant en oeuvre les étapes ci-dessous consistant à :
   identifier (216) l'ensemble de signaux $S_k$ comme un ensemble le plus prédictif de signaux $S_k$ parmi un ensemble de signaux $s_1, s_2, ..., s_D$ pour chacun d'un ou plusieurs résultats $o_k$ ;
   mettre en oeuvre un apprentissage autonome (220) de l'ensemble de modèles prédictifs probabilistes $\hat{o}_k = M_k(S_k)$, où $\hat{o}_k$ est une prédiction de résultat $o_k$ dérivée du modèle $M_k$ qui utilise, en tant qu'entrées, des valeurs obtenues à partir de l'ensemble de signaux $S_k$ ;
   répéter l'étape d'apprentissage autonome (220) de manière incrémentielle à partir de données qui contiennent des exemples de valeurs de signaux $s_1, s_2, ..., s_D$ et de résultats correspondants $o_1, o_2, ..., o_K$ ; et
   construire (214) le modèle prédictif à partir des signaux et des résultats correspondants.

2. Procédé selon la revendication 1, dans lequel l'apprentissage autonome (220) de l'ensemble de modèles prédictifs probabilistes comprend l'utilisation d'une structure de modèle linéaire en vue d'identifier des variables prédictives pour chaque incrément de données.

3. Procédé selon la revendication 2, dans lequel la structure de modèle linéaire est construite avec la forme $\hat{o}_k = f_k\left(a_0 + \sum_{i=1}^{d} a_i s_i\right)$, où $f_k$ est une fonction de mise en correspondance mettant en correspondance une entrée sur une sortie, et où $\alpha_0, \alpha_1, ..., \alpha_d$ sont des coefficients de modèle linéaire.

4. Procédé selon l'une quelconque des revendications 1, 2, ou 3, dans lequel :

   le seuil physiologique est un volume d'hémorragie ;
   le modèle prédictif est un modèle de compensation hémodynamique ;
   la détermination d'un seuil physiologique comprend l'application du modèle de compensation hémodynamique aux données physiologiques en temps réel et/ou aux données de fonctions physiologiques dérivées, en vue de prédire le volume d'hémorragie aigüe du patient ; et
   le modèle de compensation hémodynamique est généré à partir des données historiques, les données historiques comprenant une pluralité de valeurs de données collectées auprès de capteurs physiologiques fixés à une pluralité de sujets.

5. Procédé selon l'une quelconque des revendications 1, 2 ou 3, dans lequel :

   le seuil physiologique est un volume d'hémorragie aigüe d'un patient qui engendrera une défaillance cardiovasculaire ;
   le modèle prédictif est un modèle de compensation hémodynamique ;

EP 2 356 579 B1

la détermination du seuil physiologique comprend l'application du modèle de compensation hémodynamique aux données physiologiques en temps réel et/ou aux données de fonctions physiologiques dérivées, en vue de prédire le volume d'hémorragie aigüe du patient, qui engendrera une défaillance cardiovasculaire ; et le modèle de compensation hémodynamique est généré à partir des données historiques, les données historiques comprenant une pluralité de valeurs de données précédemment collectées à partir de capteurs physiologiques fixés à une pluralité de sujets.

6. Procédé selon l'une quelconque des revendications 4 ou 5, dans lequel ledit un ou lesdits plusieurs capteurs physiologiques comportent un capteur sélectionné à partir du groupe constitué par : un dispositif de surveillance pour la pression sanguine, un dispositif de surveillance non invasif pour la pression sanguine, un électrocardiographe, un sphygmo-oxymètre, un capteur Doppler transcrânien, un cardiographe d'impédance, un finomètre, un spectromètre infrarouge, un capteur de capnographie et un photopléthysmographe.

7. Procédé selon l'une quelconque des revendications 4 ou 5, dans lequel les valeurs de données collectées comprennent des valeurs de données sélectionnées à partir du groupe constitué par : un indice de perfusion, un indice de variabilité de la pleth, un débit cardiaque, un volume de pulsation cardiaque, une pression artérielle, une pression systolique, une pression diastolique, une pression artérielle moyenne, une variabilité de pression systolique, une pression différentielle, une variabilité de pression différentielle, un débit systolique, un index cardiaque et des données de spectroscopie en infrarouge proche.

8. Procédé selon la revendication 1, comprenant en outre les étapes ci-dessous consistant à :

   dériver des secondes données de fonctions physiologiques à partir des données physiologiques ;
   déterminer un second seuil physiologique à partir des secondes données de fonctions physiologiques et de données historiques, dans lequel le second seuil physiologique correspond à un point de sorte que, lorsque les secondes données de fonctions physiologiques atteignent le second seuil physiologique, un événement physiologique différent se produit ou est détecté ; et
   fournir, au niveau d'une interface d'utilisateur, la relation entre le second seuil physiologique et les données de fonctions physiologiques, à mesure que les secondes données de fonctions physiologiques sont dérivées.

9. Procédé selon l'une quelconque des revendications 1 à 8, dans lequel la fourniture comprend la représentation graphique du seuil physiologique et des données de fonctions physiologiques en fonction du temps.

10. Procédé selon l'une quelconque des revendications 1 à 9, dans lequel les données physiologiques comprennent des données sélectionnées à partir de la liste constituée par : des données de pression sanguine, des données d'électroencéphalogramme, des données de fréquence cardiaque, des données de sang désoxygéné, des données de sang oxygéné, une activité musculaire et une inhalation d'oxygène.

11. Procédé selon l'une quelconque des revendications 1 à 10, dans lequel les données de fonctions physiologiques comprennent des données sélectionnées à partir de la liste constituée par : une pression systolique, une pression artérielle, une pression artérielle moyenne, une variabilité de pression différentielle, un débit systolique, un débit cardiaque, un index cardiaque, une variabilité de pression systolique, et une pression diastolique.

12. Procédé selon l'une quelconque des revendications 1 à 11, comprenant en outre la détermination d'une réponse physiologique à un traitement, par la surveillance de la convergence ou de la divergence du seuil physiologique et des données de fonctions physiologiques en fonction du temps.

13. Système destiné à prédire des phénomènes physiologiques, le système comprenant :

   un processeur (102) et un support de stockage lisible par ordinateur (110b), le support de stockage lisible par ordinateur (110b) comprenant :

   des instructions pour recevoir des données physiologiques en temps réel en provenance d'un capteur physiologique (130) qui mesure une caractéristique physiologique d'un patient ;
   des instructions pour dériver des données de fonctions physiologiques à partir des données physiologiques ;

   **caractérisé par** :

des instructions pour déterminer, à partir d'un modèle prédictif, un seuil physiologique à partir des données de fonctions physiologiques et de données historiques, dans lequel le seuil physiologique correspond à un point de sorte que, lorsque les données de fonctions physiologiques atteignent le seuil physiologique, une anomalie physiologique est considérée comme étant présente, le modèle prédictif comprenant un ensemble de modèles prédictifs probabilistes $\hat{o}_k = M_k (S_k)$, où $\hat{o}_k$ est une prédiction de résultat $o_k$ dérivée du modèle $M_k$ qui utilise, en tant qu'entrées, des valeurs obtenues à partir d'un ensemble de signaux $S_k$ dérivé en utilisant les données physiologiques en provenance du capteur physiologique (130, 204-1, 304-2, 304-3), dans lequel les instructions pour déterminer le seuil physiologique à partir des données de fonctions physiologiques comportent :

des instructions pour identifier l'ensemble de signaux $S_k$ comme un ensemble le plus prédictif de signaux $S_k$ parmi un ensemble de signaux $s_1, s_2, ..., s_D$ pour chacun d'un ou plusieurs résultats $o_k$ ;
des instructions pour mettre en oeuvre un apprentissage autonome de l'ensemble de modèles prédictifs probabilistes $\hat{o}_k = M_k (S_k)$, où $\hat{o}_k$ est une prédiction de résultat $o_k$ dérivée du modèle $M_k$ qui utilise, en tant qu'entrées, des valeurs obtenues à partir de l'ensemble de signaux $S_k$;
des instructions pour répéter l'étape d'apprentissage autonome de manière incrémentielle à partir de données qui contiennent des exemples de valeurs de signaux $s_1, s_2, ..., s_D$ et de résultats correspondants $o_1, o_2, ..., o_K$; et
des instructions pour construire le modèle prédictif à partir des signaux et des résultats correspondants ; et

des instructions pour fournir, au niveau d'une interface d'utilisateur (106), la relation entre le seuil physiologique et les données de fonctions physiologiques à mesure que des données de fonctions physiologiques sont dérivées des données physiologiques.

FIG. 1

FIG. 2

*FIG. 3*

Read Values of Physiological Sensors from Subject　404

Generate Hemodynamic Compensation Model from Data　408

Apply Hemodynamic Compensation Model to Make Patient-Specific Predictions　412

Display Results of Predictions　416

Archive Results for Future Model Building and Prediction　420

400

*FIG. 4*

*FIG. 5*

*FIG. 6*

1105

1120

Actual LNPB
Predicted LNPB
Predicted CV Collapse

*FIG.7*

1110

1115

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003125612 A

**Non-patent literature cited in the description**

- **SHOEMAKER et al.** Outcome prediction of emergency patients by noninvasive hemodynamic monitoring. *Chest,* 2001

- **PROCOPIO et al.** Learning in Dynamic Environments with Ensemble Selection for Outdoor Robot Navigation. *the 2008 IEEE conference on intelligent robots and systems,* 22 September 2008, 620-627